(19) **Europäisches Patentamt / European Patent Office / Office européen des brevets**

(11) **EP 1 847 275 A1**

(12) # EUROPEAN PATENT APPLICATION
published in accordance with Art. 158(3) EPC

(43) Date of publication:
**24.10.2007 Bulletin 2007/43**

(51) Int Cl.:
*A61K 49/00* (2006.01)    *A61K 49/22* (2006.01)
*A61K 33/00* (2006.01)

(21) Application number: **05782127.4**

(22) Date of filing: **30.08.2005**

(86) International application number:
**PCT/CN2005/001361**

(87) International publication number:
**WO 2006/072197 (13.07.2006 Gazette 2006/28)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.01.2005 CN 200510000344**

(71) Applicant: **CHONGQING HAIFU(HIFU) TECHNOLOGY CO., LTD**
**Chongqing 401121 (CN)**

(72) Inventors:
• **WANG, Zhibiao**
  **Yubei District, Chongqing 401121 (CN)**
• **LI, Faqi**
  **Yubei District, Chongqing 401121 (CN)**
• **LIU, Liping**
  **Yubei District, Chongqing 401121 (CN)**
• **XIAO, Yanbing**
  **Yubei District, Chongqing 401121 (CN)**
• **XIAO, Ziwen**
  **Yubei District, Chongqing 401121 (CN)**
• **WANG, Zhilong**
  **Yubei District, Chongqing 401121 (CN)**

(74) Representative: **Kampfenkel, Klaus et al**
**Blumbach - Zinngrebe**
**Patentanwälte**
**Alexandrastrasse 5**
**65187 Wiesbaden (DE)**

(54) **A HIGH-INTENSITY FOCUSED ULTRASOUND PLASMID ADJUVANT AND ITS USE**

(57) The present invention discloses a plasmid enhancement agent for high intensity focused ultrasound (HIFU) treatment, which can increase acoustic energy deposition at the target location during HIFU treatment. The enhancement agent comprises a nanometer-sized biocompatible solid. The present invention also discloses another plasmid enhancement agent for HIFU treatment, wherein, the enhancement agent comprises a discontinuous phase is comprised of a core material encapsulated by a membrane-forming material, and a continuous phase comprised of aqueous medium; the discontinuous phase is uniformly dispersed in the continuous phase and has a particle size ranging from 10-1000nm; the amount of the membrane-forming material in the enhancement agent is 0.1-100g/L; and the core material comprises nanometer-sized biocompatible solid selected from the group consisting of magnetic biomaterials, hydroxylapatite, and calcium carbonate, and the amount of the core material in the enhancement agent is 0.1-150g/L. The plasmid enhancement agent for HIFU treatment of the present invention can significantly change the acoustic environment of the target location and can increase acoustic energy deposition at the target location during HIFU treatment. Eventually, the effectiveness of clinical HIFU treatment to ablate the tumor cells can be significantly improved. Accordingly, the present invention discloses use of the above plasmid enhancement agent for HIFU treatment during HIFU treatment.

**EP 1 847 275 A1**

**Description**

**FIELD OF THE PRESENT INVENTION**

The present invention is related to the fields of medicine and medical treatment, specifically to the field of ultrasound treatment, and more particularly to a plasmid enhancement agent for HIFU treatment, which can increase acoustic energy deposition to target locations during HIFU treatment, and use thereof.

**BACKGROUND OF THE PRESENT INVENTION**

High-intensity focused ultrasound (HIFU) as a new technique to treat tumors and other diseases has already been recognized in clinical applications. HIFU employs focused ultrasound, which provides continuous, high-intensity ultrasound energy at the focus, resulting in instantaneous thermal effects (65-100°C), cavitation effects, mechanical effects and sonochemical effects, to selectively cause coagulative necrosis at the focus and prevent tumors from proliferation, invasion and metastasis.

It was demonstrated that the acoustic energy was attenuated exponentially as the transmission distance increased during ultrasound transmission within the body (Baoqin Liu et al., Chinese Journal of Ultrasound in Medicine, 2002, 18(8):565-568). In addition, the energy during ultrasound transmission in soft tissues was attenuated due to tissue absorption, scattering, refraction, diffraction and the like, among which the tissue absorption and scattering are mainly responsible for the energy loss (Ruo Feng and Zhibiao Wang as editors in chief, Practical Ultrasound Therapeutics, Science and Technology Reference Publisher of China, Beijing, 2002.14). Therefore, when the HIFU treatment is used to treat deep-seated and large-sized tumors, the acoustic energy transmitted to the target would be relatively low. Thus, therapeutic efficiency would decrease and treatment time would be prolonged due to the acoustic energy attenuation.

Of course, although the transmitting power of the therapeutic transducer might be increased in order to improve the therapeutic efficiency, the normal tissue along the pathway of the ultrasound transmission is more likely to be burned in a high-intensity ultrasound environment.

In addition, at present, when the HIFU technique is clinically applied to a hepatic tumor that is blocked by the ribs in the pathway of the ultrasound transmission, the ribs are usually removed in order to increase the energy deposition at the target location, shorten the treatment time and improve therapeutic effects. Thus the noninvasiveness of HIFU treatment cannot be ensured, which is undesirable for the patients and doctors.

The above problems have disadvantageously limited the use of the HIFU treatment as a technique for clinical practice. Therefore, the technical problems with respect to increasing the energy deposition at target location, effectively treating the deep-seated tumors without damaging the surrounding normal tissue in the acoustic pathway, and treating a hepatic tumor that is blocked by the ribs without removal of the ribs, need to be solved urgently.

**SUMMARY OF THE INVENTION**

One objective of the present invention is to provide a plasmid enhancement agent for HIFU treatment, which can enhance the acoustic energy deposition at target tissue during HIFU treatment.

Another objective of the present invention is to provide a method for enhancing acoustic energy deposition at the target location during HIFU treatment using the plasmid enhancement agent for high intensity focused ultrasound (HIFU) treatment of the present invention.

A further objective of the present invention is to provide use of a plasmid enhancement agent for HIFU treatment to enhance the effectiveness of HIFU treatment.

In order to achieve the above objectives, in one embodiment, the present invention provides a plasmid enhancement agent for HIFU treatment. The enhancement agent of the present invention is a substance that can enhance acoustic energy absorption at the target location to be treated with HIFU after its administration to a biological body, i.e. a substance that can be used to reduce the acoustic energy needed to cause lesions of a target tissue (tumor and non-tumor tissue) during HIFU treatment. In the present invention, the types of substances used as enhancement agents for HIFU treatment are not particularly limited, as long as the substances are nanometer-sized biocompatible solids and can change the acoustic environment of the target tissue and promote therapeutic acoustic energy absorption and deposition at the target tissue.

Specifically, the enhancement agent for HIFU treatment of the present invention comprises preferably the nanometer-sized biocompatible solids selected from a group consisting of magnetic biomaterials, such as superparamagnetic iron oxide (SPIO), hydroxylapatite (HAP) and calcium carbonate, preferably hydroxylapatite. The enhancement agent has a particle size ranging from 1nm-500nm, preferably 1-200nm, and more preferably 10-100nm.

The method for preparing the enhancement agent for HIFU treatment is not particularly limited. For example, the aforementioned nanometer-sized biocompatible solid (such as magnetic biomaterials, such as superparamagnetic iron oxide (SPIO), hydroxylapatite (HAP) and calcium carbonate) having a, desired particle size can be added to an aqueous medium to form a suspension with a concentration of 0.1-150g/L. Prior to use, it is preferable to homogenize the suspension with a mixing device, such as a sonicator, so that the nanometer-sized biocompatible solid can be completely and homogeneously dispersed in the aqueous suspending medium.

As used herein, the term of "nanometer-sized" refers to a particle size of more than 1nm and less than 1000nm.

In order to prevent the nanometer-sized biocompatible solids from agglomeration or precipitation in the aqueous medium, a preferred embodiment of the present invention provides an enhancement agent for HIFU treatment that comprises a discontinuous phase comprised of a core encapsulated by a membrane-forming material, and a continuous phase comprised of aqueous medium. The discontinuous phase is uniformly dispersed in the continuous phase and has a particle size ranging from 10-1000nm, preferably 10-500nm and more preferably 10-200nm. The amount of the membrane-forming material in the enhancement agent is 0.1-100g/L, preferably 0.5-20g/L and more preferably 0.5-10g/L. The nanometer-sized biocompatible solids are used as the core material, and are selected from the group consisting of magnetic biomaterials, such as superparamagnetic iron oxide (SPIO), hydroxylapatite (HAP), and calcium carbonate, preferably hydroxylapatite. The particle size of the nanometer-sized biocompatible solid is 1-500nm, preferably 1-200nm, and more preferably 10-100nm. The amount of the core material in the enhancement agent is 0.1-150g/L, preferably 10-100g/L, and more preferably 20-80g/L.

In the above embodiment of the present invention, the membrane-forming material includes: lipids, such as 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine, cholesterol, and glycolipide; saccharides, including, for example, glucose, fructose, sucrose, starch and the degradation products thereof; proteins, such as albumin, globulin, fibrinogen, fibrin, hemoglobin, and the degradation products of plant proteins and the like.

In the above embodiment of the present invention, the aqueous medium is distilled water, physiological saline or glucose solution. The concentration of the glucose solution can be up to 50% (w/v). However, the glucose solution cannot be used as the aqueous medium for the plasmid enhancement agent for HIFU treatment in diabetic patients.

In a preferred embodiment, the enhancement agent may also contain carboxymethylcellulose sodium(CMC-Na) and/or glycerin. The amount of the CMC-Na in the enhancement agent is 0.01-1g/L, preferably 0.05-0.6g/L, and more preferably 0.1-0.3g/L. The amount of glycerin in the enhancement agent is 5-100g/L. The enhancement agent may also contain carboxymethylcellulose potassium, carboxyethylcellulose sodium, carboxyethylcellulose potassium, carboxypropylcellulose sodium, carboxypropylcellulose potassium, and the like.

In a more preferred embodiment, in order to increase the stability of the enhancement agent, the enhancement agent is adjusted to pH 3.0-6.5, preferably 5.0-6.0. Inorganic or organic acids may be used to adjust the pH value of the enhancement agent. Acetic acid is preferably used to adjust the pH value of the enhancement agent.

Additionally, in order to make the plasmid enhancement agent for HIFU treatment according, to the present invention target a specific tumor tissue or focus, substances having specific affinity to the tumor tissue or the focus, such as a tumor-specific antibody, may be added into the enhancement agent.

In the plasmid enhancement agent for HIFU treatment of the present invention, it is preferable to use lipid (more preferably phospholipin) to encapsulate the nanometer-sized magnetic biomaterials, hydroxylapatite (HAP) and/or calcium carbonate, such that the enhancement agent can be injected intravenously, transported through the blood circulation smoothly, and then phagocytosed quickly by the tissues of the human body, which are full of reticuloendothelial cells. Therefore, a mass of enhancement agent can be deposited in the tissues of the human body in a short time, significantly changing the acoustic environment of the target tissue, then the ultrasound absorption capacity of the tissue can be significantly enhanced, the acoustic energy deposition at the target tissue during HIFU treatment can be increased, and eventually the effectiveness of clinical HIFU treatment to ablate the tumor cells can be improved greatly.

The nanometer-sized biocompatible solids as used in the present invention are commercially available. Alternatively, the biocompatible solids may be processed into nanometer-sized granules using methods known by a skilled person in the art.

The nanometer-sized biocompatible solids encapsulated by a membrane according to the present invention may be prepared as follows:

(1) weighing and mixing a membrane-forming material and nanometer-sized biocompatible solids to obtain 0.1-100g/L membrane-forming material and 0.1-150g/L nanometer-sized biocompatible solids, adding an aqueous medium to the mixture up to a predetermined volume and stirring to form a liquid mixture;
(2) placing the liquid mixture prepared in step (1) in a sonicator and sonicating the liquid mixture for 2 to 3 minutes at a power of 400W to 800W to form a uniformly dispersed, stable suspension. Preferably, the suspension was sonicated twice to obtain a more uniformly dispersed, stable suspension.

In the method for preparing the plasmid enhancement agent for HIFU treatment of the present invention, carboxymethylcellulose sodium and/or glycerin are preferably added into the mixture before adding of aqueous medium in the step (1). More preferably, after the mixture is added with the aqueous medium and evenly stirred in the step (1), acetic acid is added to adjust the liquid mixture to pH 3.0-6.5 and preferably 5.0-6.0.

In the method for preparing the plasmid enhancement agent for HIFU treatment of the present invention, it only needs to process the biocompatible solids into nanometer-sized granules. The uniform dispersion of the discontinuous phase in the aqueous medium can be achieved simply by using a sonicator, meanwhile preventing the nanometer-sized granules from conglomerating. Thus, there are fewer requirements for the equipment used in the method, and the method is simple to use, effective for uniform dispersion, and cost-effective.

The present invention is further directed to a method for increasing energy deposition at the target location during the HIFU treatment; the method comprises administering an effective dosage of the plasmid enhancement agent of the present invention intravenously via continuous and rapid IV instillation or bolus injection to a patient at 0-168h before applying HIFU treatment to a patient. The effective dosage mentioned above varies with the type of tumor, weight of patient, location of tumor, volume of tumor and the like. However, a doctor or a pharmacist can easily determine the suitable dosage for different patients. For example, the dosage can be selected from the range of 0.1-10ml/kg, preferably 0.1-5ml/kg, and more preferably 0.5-5ml/kg.

As used herein, the term "lesion" refers to the substantial change in the physiological state of a tumor or normal tissue, generally refers to the coagulative necrosis of a tumor or normal tissue. Energy efficiency factor (EEF) can be used to quantify the acoustic energy needed to cause lesions of a target tissue per unit volume of the tissue. EEF is presented by the expression of $EEF = \eta Pt \big/ V$ (unit: J/mm$^3$), and refers to the acoustic energy needed to cause lesions of a tumor or normal tissue per unit volume of the tissue, wherein, $\eta$ refers to the focusing coefficient of a HIFU transducer, which reflects the ultrasound, energy focusing capacity of the transducer, here $\eta=0.7$; P refers to the total acoustic power of a HIFU source (unit: W), t refers to the total time of HIFU treatment (unit: s); and V refers to the volume of HIFU-induced lesions (unit: mm$^3$). A substance that greatly decreases the EEF of the target tissue after its administration is more suitable to be used as the enhancement agent for HIFU treatment.

The enhancement agent for HIFU treatment greatly decreases the EEF of the target tissue after its administration. As a result, the ratio between the EEF of the target tissue measured before the administration of the enhancement agent (i.e. $EEF_{(base)}$) and the EEF of the target tissue measured after the administration of the enhancement agent (i.e. $EEF_{(measurement)}$) is more than 1, preferably more than 2, and more preferably over 4. The upper limit of the ratio is not particularly limited and higher ratio is preferred.

## DETAILED DESCRIPTION OF THE INVENTION

Example 1

The following materials were mixed: 2.5g HAP with a particle size ranging from 1nm to 100nm (purchased from the Engineering Research Center for Biomaterials of Sichuan University), 0.3g yolk lecithin for injection (purchased from Shanghai Chemical Reagent Company) and 0.3g CMC-Na (purchased from Shanghai Chemical Reagent Company), and distilled water to a final volume of 100ml. After being uniformly mixed, the mixture was adjusted with acetic acid to pH 5.0. The mixture was sonicated for 2 minutes at a power of 400W with the transmitter of the sonicator positioned under the surface of the mixture at 1.5cm depth. After sonication, a milk-white, uniformly dispersed, stable suspension was obtained. The particle size of the discontinuous phase of the resulting enhancement agent ranged from 10nm to 1000nm, with an average range of 100nm to 500nm.

Example 2

The following materials were mixed: 2.5g HAP with a particle size ranging from 1nm to 100nm (purchased from the Engineering Research Center for Biomaterials of Sichuan University), 0.3g yolk lecithin for injection (purchased from Shanghai Chemical Reagent Company) and 1ml glycerin for injection, and distilled water to a final volume of 100ml. After being uniformly mixed, the mixture was adjusted with acetic acid to pH 5.0. The mixture was sonicated for 2 minutes at a power of 400W with the transmitter of the sonicator positioned under the surface of the mixture at 1.5cm depth. After sonication, a milk-white, uniformly dispersed, stable suspension was obtained. The particle size of the discontinuous phase of the resulting enhancement agent ranged from 10nm to 1000nm, with an average range of 100nm to 500nm.

Examples 3-5

The plasmid enhancement agent for HIFU treatment of the present invention was prepared according to the same method and procedures described in Example 1 with the materials and the amounts set forth in Table 1. The parameters of the products are also shown in Table 1.

Table 1

| | Example 3 | Example 4 | Example 5 |
|---|---|---|---|
| Nnanometer-sized HAP (particle size) | 25g/L (1-500nm) | 25g/L (1-500nm) | 50g/L (1-500nm) |
| Lecithin | 0.3g | 0.3g | 0.6g |
| CMC-Na | 0.3g | 0.6g | 0.3g |
| Glycerin for injection | 1ml | 1ml | 2ml |
| Final volume after distilled water added | 100ml | 100ml | 100ml |
| PH (c.a.) | 5.0 | 5.0 | 5.0 |
| Particle size of the discontinuous phase | 10-1000nm | 10-1000nm | 10-1000nm |
| Osmotic pressure (mosm/ kg. H2O) | 275 (Isosmotic) | 275 (Isosmotic) | 275 (Isosmotic) |

Example 6

The nanometer-sized hydroxylapatite (HAP) purchased from the Engineering Research Center for Biomaterials of Sichuan University was white powder with a particle size ranging from 10nm to 200nm with a normal distribution. HAP was dissolved in a 9% physiological saline solution to obtain two milk-white suspensions with concentrations of 25g/L and 50g/L, respectively. Prior to use, the suspensions were sonicated for 2 minutes by a sonicator under a power of 600W so as to be uniformly dispersed.

Animal test 1 Combined use of the enhancement agent as prepared in Example 1 and HIFU therapeutic devices Model JC

Thirty-six New Zealand white rabbits weighing approximately 2kg, which were provided by the Laboratory Animals Center of Chongqing University of Medical Sciences, were randomly divided into one control group and two experimental groups using the HIFU enhancement agent, 12 rabbits for each group. The rabbits in the control group were administered with physiological saline solution (2ml/kg) by rapid injection via rabbit ear border vein. The rabbits in the experimental groups were administered with the agent as prepared in Example 1 (2ml/kg) by rapid injection via rabbit ear border vein and then flushed with 1ml physiological saline solution in order to ensure that the agent had entered into the body completely. Two experimental groups were exposed with HIFU at 24 hours and 48 hours after injection of agent, respectively. The group that was exposed to HIFU at 24 hours after injection was called the first experimental group and the group that was exposed HIFU 48 hours after injection was called the second experimental group. A High-intensity Focused Ultrasound Tumor Therapeutic System Model-JC manufactured by Chongqing Haifu (HIFU) Technology Co. Ltd. was used to radiate the livers of the rabbits in the control group and two experimental groups under single pulse exposure. The High-intensity Focused Ultrasound Tumor Therapeutic System Model-JC was composed of an adjustable power generator, a B-mode ultrasound monitoring system, a therapeutic transducer, a mechanical motion control system, a treatment bed, and an acoustic coupling device. The therapeutic transducer of the system, with a working frequency of 1MHz, diameter of 150mm and focal distance of 150mm, using standard circulating degassed water with gas content of less than or equal to 3ppm, can produce a focal region of $2.3 \times 2.4 \times 26$mm and deliver an average acoustic intensity of 5500W/cm$^2$. In this test, the acoustic power for exposure was 220W, the frequency was 1.0MHZ, the exposure depth was 20mm, and the exposure duration was 15 seconds. The animals were sacrificed and dissected after HIFU exposure. The dimensions of coagulation necrosis at target location were measured. The EEFs needed to produce certain coagulative necrosis in the rabbit livers in the control group and two experimental groups are shown in Table 2.

Table 2

| Group | Number of exposure spots | V (mm$^3$) | EEF (J/mm$^3$) |
|---|---|---|---|
| Control group | 24 | 582.50±353.93 | 7.39±4.99 |
| First experimental group | 45 | 1281.56±884.56 | 2.71±1.29 |

(continued)

| Group | Number of exposure spots | V (mm³) | EEF (J/mm³) |
|---|---|---|---|
| Second experimental group | 17 | 1525.63±1007.46 | 2.25±1.61 |

As shown in Table 2, the volumes of coagulative necrosis induced in both of the experimental groups during HIFU treatment under the same conditions with the exposures carried out either at 24 hours or 48 hours after injection for the same exposure duration, were greater than that of the control group, and the EEF needed in the experimental groups decreased greatly in comparison with that of the control group. These data are statistically significant for the differences of the volumes of coagulative necrosis and the EEFs between the control group and the experimental groups ($P<0.05$).

Animal test 2 *In vivo* study of the plasmid enhancement agent for HIFU treatment as prepared in Example 6

Forty New Zealand white rabbits weighing averagely $2.7\pm0.3$kg with no limitation on sex, which were provided by the Laboratory Animals Center of Chongqing University of Medical Sciences, were randomly divided into three HAP groups and one control group, 10 rabbits for each group.

Twenty-four hours before HIFU treatment, each rabbit in HAP groups was administered with HAP suspensions as prepared in Example 6 varying in concentrations by rapid injection via rabbit ear border vein with a dosage of 2-3ml per 1kg body weight and the injection was finished within 5 seconds. Then they were flushed with 1ml physiological saline solution in order to ensure that the suspension had entered into the body completely. Each rabbit in the control group was administered with physiological saline solution (2ml/kg) by rapid injection via rabbit ear border vein. The rabbits were denuded with 8% sodium sulfide on the right bosom and abdomen. The rabbits were anesthetized by an intramuscular injection of Sumianxin (0.2ml/kg), an anesthetic agent, prior to HIFU treatment, and the abdomen wall was incised under aseptic conditions to fully expose the liver.

HIFU gynaecological therapeutic apparatus CZF-1 manufactured by Chongqing Haifu (HIFU) Technology Co. Ltd. was used to radiate the rabbit livers. The HIFU gynaecological therapeutic apparatus CZF-1 was composed of a power source, an applicator and the circulating water as disclosed in Chinese Patent No. 01144259.X. The parameters in this test were set up as follows: frequency: 9.85MHz, power: 5W, focal distance: 4mm, and treatment mode: single pulse exposure. Three exposure spots, for one cycle and 2 or 3 exposure cycles for each liver were introduced. The exposure duration was 10 seconds. The incision was sutured after HIFU treatment. Twenty-four hours later, the rabbits were sacrificed by fast injection of 10ml air via rabbit ear border vein. The dimensions of coagulation necrosis formed at target location were measured and the EEF was calculated.

T-test and relative analysis were used for comparisons between groups.

Dot-shaped gray-white coagulative necrosis appeared in the treated region immediately after HIFU treatment, and the boundary between the tissue lesions and the normal one was clear. After the rabbits in these groups were exposed to HIFU for the same duration, it could be found that the volume of focal regions (coagulative necrosis) formed in the HAP groups with different HAP dosages was greater than that in the control group administered with physiological saline solution; the EEF needed in HAP groups decreased greatly in comparison with the control group, and the difference between the HAP groups and the control group was statistically significant ($P<0.001$). By comparing the different HAP groups with different nanometer-sized HAP dosages, it is shown that when the dosage of HAP was increased, the volume of focal regions (coagulative necrosis) formed was increased greatly; the EEF needed in HAP groups decreased greatly, which was statistically significant ($P<0.001$). Table 3 shows the volume of focal regions (coagulative necrosis) and the EEFs in the HAP groups with different HAP dosages ($\bar{x}\pm s$).

Table 3

| Group | Dosage | n | V/mm³ | EEF |
|---|---|---|---|---|
| Control group | 2ml/kg | 30 | 95.3±21.6 | 0.39±0.09 |
| HAP group 1 | 50mg/kg | 30 | 153.1±41.8 | 0.24±0.05 |
| HAP group 2 | 100mg/kg | 25 | 223.2±55.1 | 0.19±0.01 |
| HAP group 3 | 150mg/kg | 21 | 287.7±47.9 | 0.13±0.00 |

Note: "n" in the table refers to the number of the exposure spots.

From the study above, it could be concluded that nanometer-sized HAP can greatly enhance the therapeutic effects of HIFU *in vivo* and that the HIFU treatment was more effective when more HAP dosage was applied.

Animal test 3 *In vitro* study of the plasmid enhancement agent for HIFU treatment as prepared in Example 6

Eighty healthy New Zealand rabbits weighing 2.5±0.3kg with no limitation on sex, which were provided by the Laboratory Animals Center of Chongqing University of Medical Sciences, were fasted for 24 hours before HIFU treatment. Then, each rabbit was administered with HAP milk-white suspension with a concentration of 25g/L as prepared in Example 6 (2ml/kg) by rapid injection via rabbit ear border vein and flushed with 1ml physiological saline solution. The rabbit livers were scanned with HIFU at 24 hours after administration of HAP (20 rabbits), 48 hours (25 rabbits), 72 hours (10 rabbits) and 168 hours (15 rabbits), respectively. Ten rabbits in the control group were administered with physiological saline solution (2ml/kg) and the rabbit livers were scanned with HIFU at 24 hours after the administration of physiological saline solution. Prior to the HIFU treatment, these rabbits were denuded with 8% sodium sulfide on the right bosom and abdomen. The rabbits were anesthetized with an intramuscular injection of Sumianxin(0.2ml/kg), and secured to a High-intensity Focused Ultrasound Tumor Therapeutic System Model JC-A.

The High-intensity Focused Ultrasound Tumor Therapeutic System Model JC-A was manufactured by the Institute of Ultrasound Engineering in Medicine, Chongqing University of Medical Sciences, and its manufacture was approved by the State Food and Drug Administration in China with the registration No. 99-301032. This system consists of a real time ultrasound monitoring and positioning apparatus and a therapeutic apparatus. The circulating degassed water was used as the acoustic coupling agent, which contained a gas of less than $3 \times 10^{-6}$. Therapeutic parameters were set up as follows: power: 220W, frequency: 1MHz, focal distance: 150mm and focal region: $2.3 \times 2.4 \times 26$mm. The therapeutic applicator was free to move in the x, y, and z directions.

The bosom and abdomen of each rabbit was immersed in the circulating degassed water and the rabbit liver was imaged clearly under B-mode ultrasound. One or two exposure spots was introduced on each liver under single pulse exposure. Each exposure spot was introduced for a fixed exposure period of 15s with an exposure depth of 20mm. Then, the rabbits were sacrificed by fast injection of 10ml air via rabbit ear border vein at 24 hours after HIFU treatment, and the liver was exteriorized and incised along the acoustic pathway, showing the location of maximum coagulative necrosis area. Then the shape of the coagulative necrosis area was determined, and the dimensions of the coagulative necrosis area as determined by TTC-staining were measured. Then the EEF was calculated.

T-test and relative analysis were used for comparisons between groups.

Under the same treatment conditions, the coagulative necrosis area formed in the HAP groups using nanometer-sized HAP was larger than that in the control group (p<0.05); and the EEF needed for HIFU treatment in the HAP groups decreased greatly in comparison with the control group. Also, in the HAP groups, the largest coagulative necrosis area was obtained with HIFU exposure at 24 hours and 48 hours after the HAP injection, and accordingly the least EEF was needed. Data indicate that it is most effective to carry out HIFU exposure at 24 hours and 48 hours after the HAP injection. If the time to carry out HIFU exposure after the HAP injection were postponed, a smaller necrosis area would be formed. Nevertheless, even at 2 weeks after the HAP injection, it was shown that the HIFU treatment was more effective in comparison with the control group (p<0.05) (see Table 4).

Table 4 Comparisons between HAP groups performing HIFU exposure at different intervals after HAP injection and the control group

| Intervals after HAP injection | n | Average exposure period (s) | Average volume of focal region (mm³) | Average EEF |
|---|---|---|---|---|
| Control group | 16 | 15 | 546.67 | 7.39±4.99 |
| 24 hours | 57 | 15.88 | 1291.56 | 2.68±1.29 |
| 48 hours | 17 | 15.6 | 1525.63 | 2.32±1.61 |
| 72 hours | 27 | 16.15 | 1153.26 | 3.28±1.35 |
| 168 hours | 26 | 15 | 920 | 2.51±0.87 |

Note: "n" in the table refers to the actual numbers of the exposure spots.

From the experiments above, it was found that nanometer-sized HAP can greatly enhance the therapeutic effects of HIFU *in vitro,* and the HIFU treatment was most effective when the HIFU exposures were carried out at 48 to 72 hours after the HAP injection.

**INDUSTRIAL APPLICABILITY**

The plasmid enhancement agent for HIFU treatment of the present invention can change the acoustic environment of the target location greatly and thus can reduce the acoustic energy needed to cause lesions in a target tissue (tumor/non-tumor tissue) per unit volume of the tissue during HIFU treatment. Accordingly, deep-seated and large-sized tumors can be treated with HIFU treatment more effectively under a certain acoustic power without damaging the normal tissues along the acoustic pathway. The enhancement agent allows the effective application of HIFU treatment to patients with a hepatic tumor that is blocked by the ribs in therapeutic acoustic pathway without removal of the ribs.

Although the present invention has been described in connection with the preferred embodiments, it is not intended to limit the scope of the present invention by the above descriptions of the embodiments. It should be understood that various modifications and changes to which the present invention may be applicable will be readily apparent to those skilled in the art. The claims are intended to cover the scope of the present invention.

**Claims**

1. Use of a nanometer-sized biocompatible solid for preparing an enhancement agent for High-intensity focused ultrasound (HIFU) treatment.

2. The use according to claim 1, wherein the nanometer-sized biocompatible solid has a particle size ranging from 1nm to 500nm and is selected from the group consisting of magnetic biomaterials, hydroxylapatite, and calcium carbonate.

3. The use according to claim 2, wherein the nanometer-sized biocompatible solid comprises hydroxylapatite.

4. The use according to claim 2, wherein the nanometer-sized biocompatible solid has a particle size ranging from 1nm to 200nm.

5. The use according to claim 4, wherein the nanometer-sized biocompatible solid has a particle size ranging from 10nm to 100nm.

6. An enhancement agent for HIFU treatment, wherein, the enhancement agent comprises a discontinuous phase comprised of a core material encapsulated by a membrane-forming material, and a continuous phase comprised of aqueous medium; the discontinuous phase is uniformly dispersed in the continuous phase and has a particle size ranging from 10-1000nm; the amount of the membrane-forming material in the enhancement agent is 0.1-100g/L; and the core material comprises nanometer-sized biocompatible solid and the amount of the core material in the enhancement agent is 0.1-150g/L.

7. The enhancement agent according to claim 6, wherein the discontinuous phase has a particle size ranging from 10-500nm.

8. The enhancement agent according to claim 7, wherein the discontinuous phase has a particle size ranging from 10-200nm.

9. The enhancement agent according to claim 6, wherein the discontinuous phase has a particle size ranging from 1-500nm and is selected from the group consisting of magnetic biomaterials, hydroxylapatite, and calcium carbonate.

10. The enhancement agent according to claim 9, wherein the nanometer-sized biocompatible solid is comprised of hydroxylapatite.

11. The enhancement agent according to claim 9, wherein the nanometer-sized biocompatible solid has a particle size ranging from 1-200nm.

12. The enhancement agent according to claim 11, wherein the nanometer-sized biocompatible solid has a particle size ranging from 10-100nm.

13. The enhancement agent according to claim 6, wherein the membrane-forming material is selected from the group consisting of one or more substances in the group of phospholipin, cholesterol and glycolipide.

14. The enhancement agent according to claim 13, wherein the membrane-forming material comprises phospholipin selected from the group consisting of 3-sn-phosphatidylcholine, 1,2-dipalmitoyl-sn-glycero-3-phosphatidylglycerol sodium salt, 1,2-distearoyl-sn-glycero-3-phosphatidylcholine, sodium 1,2-dipalmitoyl-sn-glycero-3-phosphatidate, 1,2-dipalmitoyl-sn-glycero-3-phosphatidylcholine, phosphatidylserine and hydrogenated phosphatidylserine.

15. The enhancement agent according to claim 6, wherein the amount of the membrane-forming material in the enhancement agent is 0.5-20g/L.

16. The enhancement agent according to claim 15, wherein the amount of the membrane-forming material in the enhancement agent is 0.5-10g/L.

17. The enhancement agent according to claim 6, wherein the amount of the core material in the enhancement agent is 10-100g/L.

18. The enhancement agent according to claim 17, wherein the amount of the core material in the enhancement agent is 20-80g/L.

19. The enhancement agent according to claim 6, wherein the aqueous medium comprises distilled water, physiological saline solution or glucose solution.

20. The enhancement agent according to any one of claims 6-19, wherein the enhancement agent contains 0.01-10g/L carboxymethylcellulose sodium and/or 5-100g/L glycerin.

21. A method for increasing acoustic energy deposition at target location during HIFU treatment, wherein, the method comprises:

administering the plasmid enhancement agent according to any one of claims 1-20 in an effective dosage intravenously via continuous and rapid IV instillation or bolus injection to a patient at 0-168h before the application of HIFU treatment to the target location of a patient.

| INTERNATIONAL SEARCH REPORT | International application No. |
|---|---|
| | PCT/CN2005/001361 |

**A. CLASSIFICATION OF SUBJECT MATTER**

$ICP^7$:A61K49/00,49/22,33/00

According to International Patent Classification (IPC) or to both national classification and IPC

**B. FIELDS SEARCHED**

Minimum documentation searched (classification system followed by classification symbols)

$ICP^7$:A61K

Documentation searched other than minimum documentation to the extent that such documents are included in the fields searched

China patents, China journals

Electronic data base consulted during the international search (name of data base and, where practicable, search terms used)

WPI,PAJ,EPODOC,CPRS,CNKI,CA

Key words: hydroxyapatite, ultrasound, calcium carbonate, magnetic, lecithin, phosphatidylchloline,iodipin and the corresponding chinese words

**C. DOCUMENTS CONSIDERED TO BE RELEVANT**

| Category* | Citation of document, with indication, where appropriate, of the relevant passages | Relevant to claim No. |
|---|---|---|
| X | US4767610A(THE REGENTS OF THE UNIVERSITY OF CALIFORNIA),30.Aug.1988(30.08.1988),whole | 6-8 |
| A | US5344640A(MALLINCKRODT MEDICAL, INC.),06.Sep.1994 (06.09.1994),whole | 1-21 |

☐ Further documents are listed in the continuation of Box C.     ☒ See patent family annex.

| | |
|---|---|
| *   Special categories of cited documents: <br> "A"   document defining the general state of the art which is not considered to be of particular relevance <br> "E"   earlier application or patent but published on or after the international filing date <br> "L"   document which may throw doubts on priority claim (S) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"document member of the same patent family |

| Date of the actual completion of the international search | Date of mailing of the international search report |
|---|---|
| 10.Nov..2005(10.11.2005) | 0 8 · DEC 2005 (0 8 · 12 · 2 0 0 5) |
| Name and mailing address of the ISA/CN <br> The State Intellectual Property Office, the P.R.China <br> 6 Xitucheng Rd., Jimen Bridge, Haidian District, Beijing, China 100088 <br> Facsimile No. 86-10-62019451 | Authorized officer <br><br> Telephone No. 86-10-62085232 |

Form PCT/ISA /210 (second sheet) (April 2005)

## INTERNATIONAL SEARCH REPORT
### Information on patent family members

International application No.

PCT/CN2005/001361

| Patent Documents referred in the Report | Publication Date | Patent Family | Publication Date |
|---|---|---|---|
| US4767610A | 30.Aug.1988(30.08.1988) | none | |
| US5344640A | 06.Sep.1994(06.09.1994) | DE69231627E | 08.Feb.2001(08.02.2001) |
| | | WO9307905A2 | 29.Apr.1993(29.04.1993) |
| | | AU2886492A | 21.May1993(21.05.1993) |
| | | EP0610333A1 | 17.Aug.1994(17.08.1994) |
| | | JP7500823T | 26.Jan.1995(26.01.1995) |
| | | WO9307905A3 | 05.Aug.1993(05.08.1993) |
| | | US5468465A | 21.Nov..1995(21.11.1995) |
| | | AU674291B | 19.Dec.1996(19.12.1996) |
| | | AU7034596A | 23.Jan.1997(23.01.1997) |
| | | US5609850A | 11.Mar.1997(11.03.1997) |
| | | US5690908A | 25.Nov..1997(25.11.1997) |
| | | AU686523B | 05.Feb.1998(05.02.1998) |
| | | EP0610333B1 | 03.Jan.2001(03.01.2001). |
| | | ES2152932T3 | 16.Feb.2001(16.02.2001) |
| | | CA2120130 A1 | 29.Apr.1993(29.04.1993) |
| | | US5342609A | 30.Aug.1994(30.08.1994) |
| | | US5407659A | 18.Apr.1995(18.04.1995) |
| | | US5419892A | 30.May1995(30.05.1995) |
| | | WO9527437A1 | 19.Oct.1995(19.10.1995) |
| | | AU6766494A | 30.Oct.1995(30.10.1995) |
| | | US5560902A | 01.Oct.1996(01.10.1996) |
| | | US5595724A | 21.Jan.1997(21.01.1997) |
| | | EP0755222A1 | 29.Jan.1997(29.01.1997) |
| | | EP19940915769 | 11.Apr.1994(11.04.1994) |
| | | JP9511520T | 18.Nov..1997(18.11.1997) |
| | | AT198423T | 15.Jan.2001(15.01.2000) |
| | | DE69231627D1 | 08.Feb.2001(08.02.2001) |
| | | DE69231627T2 | 26.Apr.2001(26.04.2001) |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 01144259X

**Non-patent literature cited in the description**

- **BAOQIN LIU et al.** *Chinese Journal of Ultrasound in Medicine,* 2002, vol. 18 (8), 565-568
- **RUO FENG ; ZHIBIAO WANG.** *Practical Ultrasound Therapeutics,* 2002